# EUROPEAN PATENT APPLICATION

(11) **EP 3 913 017 A2**
(43) Date of publication of application: **24.11.2021**
(21) Application number: 21174751.4
(22) Date of filing: 19.05.2021
(51) Int. Cl.: C08L 3/02, A61F 13/15, D04H 1/413, D04H 1/425, D04H 1/587, D04H 1/732

(54) **STARCH COMPOSITE FOR BINDING FIBERS, FIBER STRUCTURE, AND FIBER STRUCTURE-MANUFACTURING APPARATUS**

(30) Priority: 21.05.2020 JP 2020088716
(71) Applicant: Seiko Epson Corporation, Tokyo 160-8801 (JP)
(72) Inventor: YOSHIOKA, Satomi, Suwa-shi, 392-8502 (JP); TANAKA, Hideki, Suwa-shi, 392-8502 (JP); YAMAGAMI, Toshiaki, Suwa-shi, 392-8502 (JP); SEKI, Shunichi, Suwa-shi, 392-8502 (JP); UENO, Yoshihiro, Suwa-shi, 392-8502 (JP); YODA, Kaneo, Suwa-shi, 392-8502 (JP); URANO, Nobutaka, Suwa-shi, 392-8502 (JP)
(74) Representative: Miller Sturt Kenyon

(57) **Abstract**

A starch composite for binding fibers includes starch particles which are first particles and second particles containing a hydrophobic material having an affinity for the starch particles, which are the first particles. The weight-average size of the second particles is less than the weight-average size of the first particles and the outer surfaces of the first particles are covered by the second particles.

## Description

The present application is based on, and claims priority from JP Application Serial Number 2020-088716, filed May 21, 2020, the disclosure of which is hereby incorporated by reference herein in its entirety.

### BACKGROUND

### 1. Technical Field

The present disclosure relates to a starch composite for binding fibers, a fiber structure, and a fiber structure-manufacturing apparatus.

### 2. Related Art

Hitherto, a sheet-manufacturing apparatus for manufacturing a sheet by adding a binder binding fibers in a dry process has been known as disclosed in JP-A-2015-92032.

In addition, JP-A-2012-144826 discloses a method in which the binder used is not a petroleum-derived material but is starch, which is a natural material.

However, starch particles are likely to absorb moisture. Therefore, there is a problem in that the surfaces of the starch particles are gelatinized and the starch particles are bound to aggregate.

The occurrence of aggregation of the starch particles reduces powder fluidity. Therefore, when starch is supplied to fibers, the supply of the starch is unstable, so that the binding strength between the fibers varies.

### SUMMARY

According to an aspect of the present disclosure, a starch composite for binding fibers includes starch particles which are first particles and second particles containing a hydrophobic material having an affinity for the starch particles, which are the first particles. The weight-average size of the second particles is less than the weight-average size of the first particles and the outer surfaces of the first particles are covered by the second particles.

According to an aspect of the present disclosure, a starch composite for binding fibers includes starch particles and coat layers which are made of a hydrophobic material having an affinity for the starch particles and which coat the starch particles.

According to an aspect of the present disclosure, a fiber structure contains cotton-like fibers, starch particles, and a hydrophobic material having an affinity for the starch particles. The starch particles and the hydrophobic material are dispersed between the cotton-like fibers and the cotton-like fibers, the starch particles, and the hydrophobic material are bound.

According to an aspect of the present disclosure, a fiber structure-manufacturing apparatus includes a disintegration section disintegrating a supplied fiber feedstock to produce disintegrated matter, an incorporation section incorporating a starch composite having a surface covered by a hydrophobic material having an affinity for starch particles into the produced disintegrated matter, a fibrous web-forming section forming a fibrous web by accumulating a mixture containing the starch composite, and a fiber structure-forming section forming a fiber structure by heating and pressing the fibrous web.

### BRIEF DESCRIPTION OF THE DRAWINGS

FIG. 1A is a schematic view illustrating the configuration of a starch composite according to an embodiment of the present disclosure.
FIG. 1B is a schematic view illustrating the configuration of another starch composite according to an embodiment of the present disclosure.
FIG. 2 is a schematic view illustrating the configuration of a fiber structure according to an embodiment of the present disclosure.
FIG. 3 is a schematic view illustrating the configuration of a fiber structure-manufacturing apparatus according to an embodiment of the present disclosure.

### DESCRIPTION OF EXEMPLARY EMBODIMENTS

First, the configuration of a starch composite Sb according to an embodiment of the present disclosure is described.

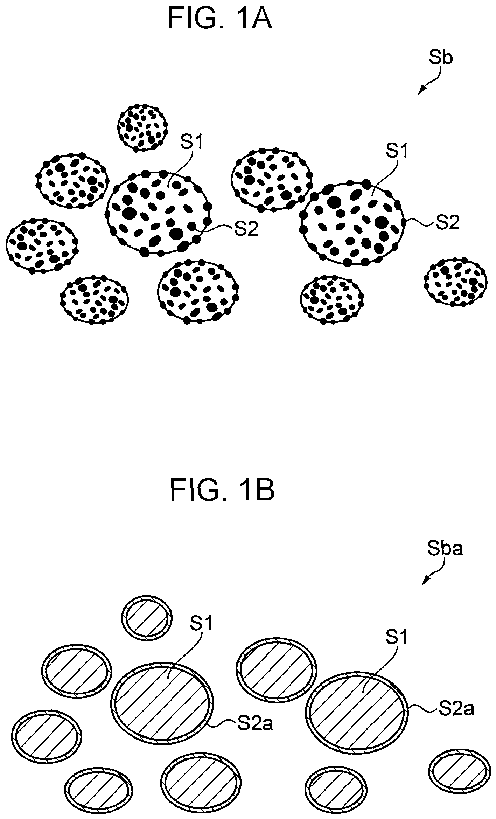

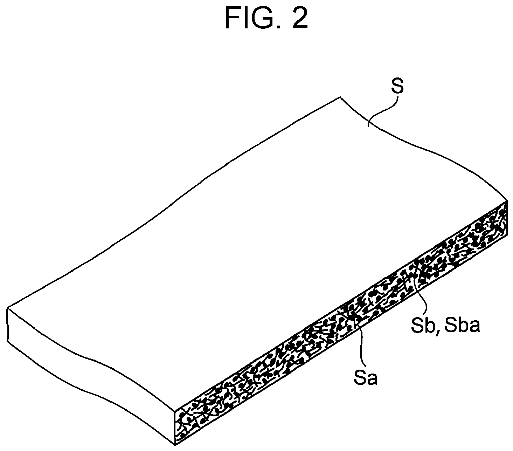

As shown in FIG. 1A, the starch composite Sb includes starch particles (first particles) S1 and second particles S2 containing a hydrophobic material having an affinity for the starch particles S1. The starch composite Sb functions as a binder for binding fibers Sa (refer to FIG. 2). The weight-average size of the second particles S2 is less than the weight-average size of the starch particles S1 and the outer surfaces of the starch particle S1 are covered by the second particles S2. In an example shown in FIG. 1A, the surfaces of the starch particles S1 are covered by the second particles S2, which are in a granular state.

In an aggregate of the starch composite Sb, the surface of each starch particle S1 need not be entirely covered by the second particles S2. When, for example, about 20% to 100% of the surface area of the starch particle S1 is covered by the second particles S2, the surface of the starch particle S1 is regarded as being covered by the second particles S2.

The starch particles S1 are particles of starch such as potato, wheat, corn sweet potato, cassava, tapioca, or rice or modified starch (for example, carboxymethyl-cellulose (CMC)). The weight-average size of the starch particles S1 is preferably 1 µm to 100 µm and more preferably 2 µm to 50 µm.

The second particles S2, which contain the hydrophobic material, are composed of one or a combination of two or more of hydrocarbons, fatty acids, fatty acid metal salts, higher alcohols, aliphatic amides, fatty acid esters, and inorganic materials. The weight-average size of the second particles S2, which contain the hydrophobic material, is preferably 0.01 µm to 1,000 µm and more preferably 0.05 µm to 20 µm. This allows the starch particles S1 to be likely to be covered by the second particles S2.

Examples of the hydrocarbons include paraffin waxes, polyethylene waxes, carnauba waxes, and rice waxes. Examples of the fatty acids include carboxylic acids such as stearic acid, rosin, shellac, and succinic acid. Examples of the fatty acid metal salts include metal stearates such as calcium stearate, magnesium stearate, zinc stearate, and lead stearate. Examples of the higher alcohols include stearyl alcohol. Examples of the aliphatic amides include stearamide, hydroxystearamide, oleamide, erucamide, methylenebisstearamide, and ethylenebisstearamide. Examples of the fatty acid esters include stearic acid monoglyceride, stearyl stearate, sugar fatty acid esters, and glycerin fatty acid esters.

Examples of the inorganic materials include mica, glass (SiO₂), calcium carbonate (CaCO₃), titanium oxide (TiO₂), and alumina.

Among the above, in particular, the fatty acids, the fatty acid metal salts, the higher alcohols, and the aliphatic amides contain a polar group and have the property of readily hydrogen-bonding to surface OH groups of the starch particles S1. Therefore, the surfaces of the starch particles S1 are likely to be covered by the second particles S2.

Herein, the surface OH groups of the starch particles S1, which are included in the starch composite Sb, have an affinity for water due to hydrogen bonding. Therefore, it is conceivable that the starch particles S1 are likely to absorb moisture, the surfaces thereof are gelatinized, and the starch particles S1 are likely to aggregate. The occurrence of aggregation of the starch particles S1 reduces powder fluidity; hence, for example, when the starch particles S1 are supplied to the fibers Sa, the supply of the starch particles S1 may possibly be unstable.

Therefore, in this embodiment, the surfaces of the starch particles S1 are covered by the second particles S2, which are hydrophobic. This inhibits the binding of the starch particles S1, thereby enabling the occurrence of aggregation of the starch particles S1 to be suppressed. Incidentally, a state in which the aggregation of the starch particles S1 is suppressed does not mean a state in which all the starch particles S1 are discrete but includes, for example, a state in which some of the starch particles S1 are aggregated. When the amount of the aggregated starch particles S1 is 10% by mass or less of the amount of the starch composite Sb, preferably about 5% by mass or less, this case is included in the state in which the aggregation of the starch particles S1 is suppressed. Furthermore, although the starch particles S1 are in contact with each other when the starch composite Sb is accommodated in a container, a bag, or the like, a case where the starch particles S1 can be made discrete by external force, such as soft stirring, dispersion by a gas flow, or free fall, insufficient to break the starch particles S1, is included in the state in which the aggregation of the starch particles S1 is suppressed.

FIG. 1B is a schematic view illustrating the configuration of another starch composite Sba according to an embodiment of the present disclosure.

The starch composite Sba includes starch particles S1 and coat layers S2a which contain a hydrophobic material having an affinity for the starch particles S1 and which coat the starch particles S1. The coat layers S2a have a thickness of, for example, 1 nm to 1 µm. The coat layers S2a have a moderately curved surface.

In an aggregate of the starch composite Sba, the surface of each of the starch particles S1 need not be entirely coated by a corresponding one of the coat layers S2a. When, for example, about 20% or more of the surface area of each of the starch particles S1 is coated by a corresponding one of the coat layers S2a, the surface of each of the starch particles S1 is regarded as being coated by a corresponding one of the coat layers S2a.

Furthermore, an aggregate of the starch composite Sba may be an aggregate in which the starch composite Sb, which is shown in FIG. 1A, is contained. That is, an aggregate of the starch composite Sb or the starch composite Sba may be an aggregate in which the starch particles S1 of which the surfaces are covered by the second particles S2 and the starch particles S1 of which the surfaces are coated by the coat layers S2a are present together.

The starch particles S1 are particles of starch such as potato, wheat, corn, sweet potato, cassava, tapioca, or rice or modified starch (for example, carboxymethyl-cellulose (CMC)). The weight-average size of the starch particles S1 is preferably 1 µm to 100 µm and more preferably 2 µm to 50 µm.

The coat layers S2a, which contains the hydrophobic material, are composed of one or a combination of two or more of hydrocarbons, fatty acids, fatty acid metal salts, higher alcohols, aliphatic amides, fatty acid esters, and inorganic materials. Incidentally, the configuration of the hydrophobic material is as described above and therefore is not described.

In the starch composite Sba, the surfaces of the starch particles S1 are coated by the coat layers S2a. This inhibits the binding of the starch particles S1, thereby enabling the occurrence of aggregation of the starch particles S1 to be suppressed.

Next, a method for producing the starch composite Sb or Sba is described. The starch composite Sb or Sba is produced by mixing the starch particles S1 and the second particles S2 (hydrophobic material). Examples of a mixing method include, but are not limited to, a method using air and a method using a high-speed rotary mixer.

In the method using air, the starch particles S1 and the second particles S2 (hydrophobic material) are charged into an airtight container. The airtight container is provided with a blowing section capable of blowing dry air into the airtight container and a valve equipped with a filter for preventing the starch particles S1 and second particles S2 in the airtight container from flowing out of the airtight container. Dry air is blown toward the starch particles S1 and second particles S2 charged into the airtight container. The starch particles S1 and the second particles S2 are mixed by the blowing pressure due to dry air. This allows starch particles S1 and the second particles S2 to attract each other, thereby forming the starch composite Sb or Sba.

In the method using the high-speed rotary mixer, for example, a known mixer capable of rotating at a high speed of 5,000 rpm to 50,000 rpm is applicable and an FM mixer, a Henschel mixer, a super mixer, or the like can be used. In the case of using the high-speed rotary mixer, the starch particles S1 and the second particles S2 are more uniformly mixed, whereby the starch composite Sb or Sba is formed.

Next, the configuration of a fiber structure S according to an embodiment of the present disclosure is described.

As shown in FIG. 2, the fiber structure S includes cotton-like fibers Sa and a starch composite Sb for binding the fibers Sa. The starch composite Sb is placed in such a state that the starch composite Sb is dispersed between the cotton-like fibers Sa. The starch composite Sb includes starch particles S1 and second particles S2 containing a hydrophobic material having an affinity for the starch particles S1. The surfaces of the starch particles S1 are covered by the second particles S2. Incidentally, the detailed configuration of the starch composite Sb is substantially the same as a configuration shown in FIG. 1A and therefore is not described.

Examples of raw materials of the fibers Sa include waste paper (for example, printed paper for indirect electrophotographic process, various types of recycled paper, corrugated fiberboard, and the like), wood pulp materials (virgin pulp (NBKP, LBKP, NUKP, LUKP, and the like)), bleached chemithermomechanical pulp (BCTMP), and synthetic pulp (for example, SWP®). These may be used alone or in combination.

The longitudinal size (fiber length) of the fibers Sa is 0.1 µm to 100 mm and is preferably 0.5 µm to 50 mm. The lateral size (fiber diameter) of the fibers Sa is 0.1 µm to 1,000 µm and is preferably 1.0 µm to 500 µm.

A method for manufacturing the fiber structure S is described.

A supplied fiber feedstock is disintegrated, whereby disintegrated matter (cotton-like fibers Sa) is produced. The starch composite Sb, in which the surfaces of the starch particles S1 are covered by the hydrophobic material (second particles S2) having an affinity for the starch particles S1, is incorporated into the disintegrated matter. A mixture containing the starch composite Sb is accumulated by an air-laid process, whereby a fibrous web is formed. The fibrous web is heated and pressed with a roller or a press machine. This allows the fiber structure S, which is sheet-shaped, to be formed.

The starch composite Sb, which is incorporated into the disintegrated matter (cotton-like fibers Sa), is one that the aggregation of the starch particles S1 is suppressed. Therefore, the starch composite Sb can bind the fibers Sa in such a state that the starch composite Sb is dispersed between the cotton-like fibers Sa. Since the aggregation of the starch particles S1 is suppressed, the separation of the starch composite Sb from the fibers Sa is reduced. That is, the starch composite Sb has enhanced supportability.

Since the surfaces of the starch particles S1 are covered by the second particles S2, which are hydrophobic, surface OH groups of the starch particles S1 are covered. This increases the difference in charge between surface OH groups of the fibers Sa and a surface of the starch composite Sb, thereby enhancing the electrostatic attraction of the starch composite Sb to surfaces of the fibers Sa. In this case, for example, in a triboelectric series, selecting the hydrophobic material that is far apart from the fibers Sa and the starch particles S1 causes a large difference in charge during friction, thereby enabling the supportability of the starch composite Sb on surfaces of the fibers Sa to be enhanced.

The configuration of the fiber structure S is a configuration equipped with the cotton-like fibers Sa and the starch composite Sb. The configuration of the fiber structure S is not limited to this configuration and may be a configuration equipped with the cotton-like fibers Sa and the starch composite Sba. Such a configuration can obtain substantially the same effect as the above. Incidentally, the configuration of the starch composite Sba is substantially the same as a configuration shown in FIG. 1B and therefore is not described.

Next, the configuration of a fiber structure-manufacturing apparatus 1 capable of manufacturing the fiber structure S is described.

The fiber structure-manufacturing apparatus 1 is an apparatus that is suitable for manufacturing the fiber structure S in such a manner that after, for example, used waste paper serving as a fiber feedstock is disintegrated into fibers in a dry mode, the fibers are pressed, are heated, and are cut. The binding strength and degree of whiteness of the fiber structure S may be enhanced or functions such as color, fragrance, and flame retardancy may be added to the fiber structure S depending on applications in such a manner that a fibrillated feedstock is mixed with various additives. For example, sheets of paper, such as A4 or A3 office paper or business card paper, having various thicknesses or sizes can be manufactured depending on applications in such a manner that the fiber structure S is formed by controlling the density, thickness, and/or shape of paper.

The fiber structure-manufacturing apparatus 1 includes a supply section 10, a rough crushing section 12, a disintegration section 20, a screening section 40, a first web-forming section 45, a rotator 49, a mixing section 50 (incorporation section), an accumulation section 60, a second web-forming section 70 (fibrous web-forming section), a transport section 79, a fiber structure-forming section 80, and a cutting section 90.

The fiber structure-manufacturing apparatus 1 further includes, for example, humidification sections 202, 204, 206, 208, 210 and 212 for the purpose of humidifying a feedstock and a space in which the feedstock is transported. Humidification suppresses the adhesion of the feedstock due to static electricity. Each of the humidification sections 202, 204, 206, and 208 is composed of, for example, an evaporation type or hot-air evaporation type of humidifier. Each of the humidification sections 210 and 212 is composed of, for example, an ultrasonic humidifier.

The supply section 10 supplies a feedstock to the rough crushing section 12. The feedstock supplied to the rough crushing section 12 may be one containing the fibers Sa and is, for example, paper, pulp, a pulp sheet, nonwoven fabric, cloth, woven fabric, or the like. A configuration in which the fiber structure-manufacturing apparatus 1 uses waste paper as a feedstock is exemplified below. The supply section 10 includes, for example, a stacker stacking and accumulating sheets of waste paper and an automatic charger feeding the waste paper to the rough crushing section 12.

The rough crushing section 12 cuts the feedstock supplied by the supply section 10 into roughly crushed pieces with rough crushing blades 14. The rough crushing blades 14 cut the feedstock in gas such as air. The rough crushing section 12 includes, for example, a pair of the rough crushing blades 14, which nip and cut the feedstock, and a driving portion rotating the rough crushing blades 14 and may have substantially the same configuration as that of a so-called shredder. The shape and size of the roughly crushed pieces are arbitrary and may be suitable for disintegration treatment in the disintegration section 20. The rough crushing section 12 cuts the feedstock into, for example, 1 cm to several centimeters square paper pieces or paper pieces with a size of 1 cm or less. The roughly crushed pieces cut by the rough crushing section 12 are transferred to the disintegration section 20 through a pipe 2 with a chute 9 therebetween.

The disintegration section 20 disintegrates the roughly crushed pieces cut by the rough crushing section 12. In particular, the disintegration section 20 disintegrates the feedstock cut by the rough crushing section 12 to produce disintegrated matter. The term "disintegrate" as used herein refers to disentangling a feedstock containing a plurality of the bound fibers Sa one by one. The disintegration section 20 has the function of separating substances, such as resin particles, ink, toner, and a bleeding inhibitor, adhering to the feedstock from the fibers Sa.

One having passed through the disintegration section 20 is referred to as disintegrated matter. The disintegrated matter contains the disentangled fibers Sa, resin particles separated from fibers when fibers are disentangled, that is, resin particles for binding a plurality of fibers, a colorant such as ink or toner, and an additive such as a bleeding inhibitor or a paper strength additive in some cases. The shape of the disentangled disintegrated matter is a string shape or a flat string shape. The disentangled disintegrated matter may be present in such a state that the disentangled disintegrated matter is not intertwined with other disentangled fibers, that is, such a state that the disentangled disintegrated matter is independent or in such a state that the disentangled disintegrated matter is intertwined with other disentangled fibers to form aggregates, that is, such a state that the disentangled disintegrated matter forms lumps.

The disintegration section 20 performs disintegration in a dry mode. Herein, performing treatment such as disintegration in gas, such as air, rather than liquid is referred to as a dry mode. The disintegration section 20 is composed of, for example, an impeller mill. In particular, the disintegration section 20 includes a rotor rotating at high speed and a liner located outside the rotor, the rotor and the liner being not shown. The roughly crushed pieces cut by the rough crushing section 12 are interposed between the rotor and liner of the disintegration section 20 and are disintegrated. The disintegration section 20 generates a gas flow by the rotation of the rotor. The gas flow enables the disintegration section 20 to suck the feedstock, that is, the roughly crushed pieces from the pipe 2 with an inlet 22 therebetween and to transport the disintegrated matter to an outlet 24. The disintegrated matter is fed to a pipe 3 from the outlet 24 and is transferred to the screening section 40 through the pipe 3. In an illustrated example, the fiber structure-manufacturing apparatus 1 includes a disintegration blower 26 that is a gas flow generator and the disintegrated matter is transported to the screening section 40 by a gas flow generated by the disintegration blower 26.

The screening section 40 is provided with an inlet 42 into which the disintegrated matter disintegrated by the disintegration section 20 flows from the pipe 3 together with the gas flow. The screening section 40 screens the disintegrated matter introduced from the inlet 42 depending on the length of the fibers Sa. In detail, the screening section 40 screens the disintegrated matter disintegrated by the disintegration section 20 into a first screened fraction which is a portion of the disintegrated matter that is smaller than a predetermined size and a second screened fraction which is a portion of the disintegrated matter that is larger than the first screened fraction. The first screened fraction contains the fibers Sa, particles, or the like. The second screened fraction contains, for example, long fibers, undisintegrated pieces, roughly crushed pieces not sufficiently disintegrated, lumps formed by the aggregation or entanglement of disintegrated fibers, and the like.

The screening section 40 includes, for example, a drum portion 41 and a housing portion 43 housing the drum portion 41.

The drum portion 41 is a cylindrical sieve rotationally driven with a motor. The drum portion 41 includes a net and functions as a sieve. Owing to meshes of the net, the drum portion 41 screens the disintegrated matter into the first screened fraction, which is smaller than the size of openings of the net, and the second screened fraction, which is larger than the size of the openings of the net. The net of the drum portion 41 used may be, for example, a metal gauze, an expanded metal obtained by expanding a slit metal plate, or a punching metal obtained by forming holes in a metal plate with a press machine or the like.

The disintegrated matter introduced from the inlet 42 is fed into the drum portion 41 together with a gas flow, so that the first screened fraction is dropped downward through the meshes of the net by the rotation of the drum portion 41. The second screened fraction, which cannot pass through the meshes of the net, is guided to an outlet 44 by a gas flow flowing from the inlet 42 into the drum portion 41 and is fed to a pipe 8. The pipe 8 connects the inside of the drum portion 41 to the pipe 2. The second screened fraction flowing through the pipe 8 is returned to the disintegration section 20 and is disintegrated.

The first screened fraction screened by the drum portion 41 passes through the meshes of the net of the drum portion 41 to disperse in air and falls toward a mesh belt 46 of the first web-forming section 45 that is located below the drum portion 41.

The first web-forming section 45 includes the mesh belt 46, three rollers 47, and a suction portion 48. The mesh belt 46 is an endless belt, is tensioned with the rollers 47, and is moved by the motion of the rollers 47 in a direction indicated by an illustrated arrow. A surface of the mesh belt 46 is composed of a net in which openings with a predetermined size are arranged. In the first screened fraction falling from the screening section 40, fine particles passing through the meshes of the net fall below the mesh belt 46 and the fibers Sa, which cannot pass through the meshes of the net, accumulate on the mesh belt 46 and are transported in the direction of the arrow together with the mesh belt 46. The fine particles falling from the mesh belt 46 include those that are in the disintegrated matter and that have a relatively small size or low density, that is, resin particles unnecessary to bind the fibers Sa, a colorant, an additive, and the like and are removed matter not used for the manufacture of the fiber structure S by the fiber structure-manufacturing apparatus 1.

The mesh belt 46 is moved at a constant speed V1 in usual operation for manufacturing the fiber structure S. The term "usual operation" as used herein refers to operation excluding the execution of start control and stop control of the fiber structure-manufacturing apparatus 1 and, in particular, refers to a period during which the fiber structure S is manufactured by the fiber structure-manufacturing apparatus 1 so as to have desired quality.

The suction portion 48 sucks air from under the mesh belt 46. The suction portion 48 is connected to a dust collection section 27 with a pipe 23 therebetween. The dust collection section 27 is a filter or cyclone type of dust collector and separates fine particles from a gas flow. A collection blower 28 is placed downstream of the dust collection section 27. The collection blower 28 functions as a dust-collecting suction section sucking air from the dust collection section 27. Air discharged from the collection blower 28 is discharged outside the fiber structure-manufacturing apparatus 1 through a pipe 29.

In a transport path of the mesh belt 46, air containing mist is supplied downstream of the screening section 40 by the humidification section 210. Mist, which is made up of fine particles of water produced by the humidification section 210, falls toward a first web W1 to supply moisture to the first web W1. This adjusts the amount of moisture contained in the first web W1, thereby enabling the adhesion of the fibers Sa to the mesh belt 46 due to static electricity to be suppressed.

The fiber structure-manufacturing apparatus 1 includes the rotator 49. The rotator 49 divides the first web W1 accumulated on the mesh belt 46. The first web W1 is separated from the mesh belt 46 at a position where the mesh belt 46 is turned with one of the rollers 47, so that the first web W1 is divided by the rotator 49.

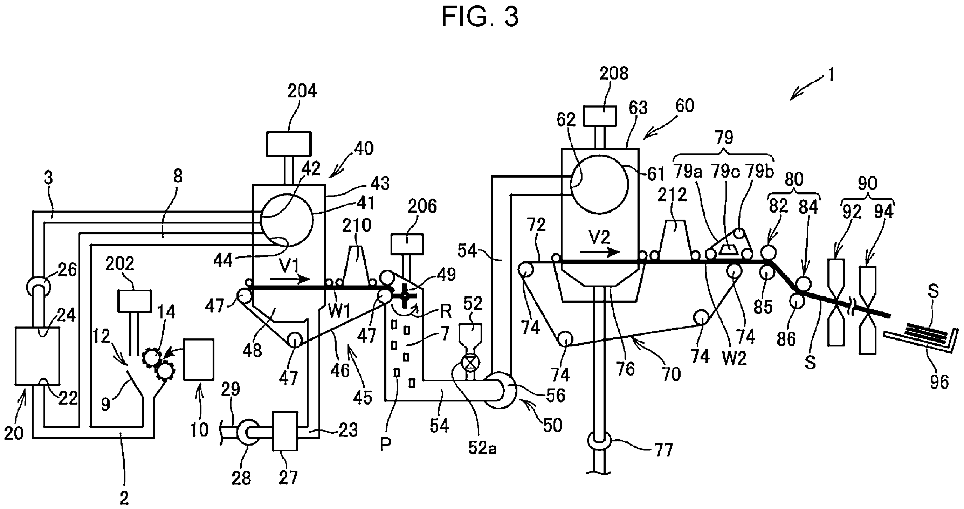

The rotator 49 includes plate-shaped vanes and has a rotary vane form. The rotator 49 is placed at a position where the first web W1 separated from the mesh belt 46 comes into contact with one of the vanes. The transported first web W1 separated from the mesh belt 46 collides with the vanes because of the rotation of the rotator 49 in, for example, a direction indicated by an arrow R in FIG. 3 and is divided, whereby fragments P are produced. The fragments P divided by the rotator 49 falls in a pipe 7 and is transported to the mixing section 50 by a gas flow flowing in the pipe 7.

The mixing section 50 incorporates the starch composite Sb into the fragments P produced by dividing the transported first web W1 and mixes the fragments P, which are composed of the fibers Sa, and the starch composite Sb together.

The mixing section 50 communicates with an accommodation section 52 accommodating the starch composite Sb and the pipe 7 and includes a pipe 54 in which the fragments P flow and a mixing blower 56.

In the mixing section 50, a gas flow is generated with the mixing blower 56 and the fragments P and the starch composite Sb are transported in the pipe 54 in such a manner that the fragments P and the starch composite Sb are mixed together. The fragments P are disintegrated into finer fibers in a course in which the fragments P flow in the pipes 7 and 54.

The accommodation section 52 is a hopper and includes a regulating valve 52a regulating the amount of the starch composite Sb supplied from the accommodation section 52 to the pipe 54.

Herein, the starch composite Sb, which is accommodated in the accommodation section 52, includes the starch particles S1 and the second particles S2, which contain the hydrophobic material having an affinity for the starch particles S1. The outer surface of each first particle S1 is covered by the second particles S2. This inhibits the binding of the starch particles S1, thereby enabling the occurrence of aggregation of the starch particles S1 to be suppressed. That is, powder fluidity is enhanced. Thus, the occurrence of failures such as a phenomenon in which the starch composite Sb accommodated in the accommodation section 52 remains on a side surface of the accommodation section 52, that is, a rathole and a phenomenon in which an upper portion of the starch composite Sb accommodated in the accommodation section 52 arches to cause a block, that is, bridging is suppressed, thereby enabling the amount of the starch composite Sb supplied to the fibers Sa to be maintained constant.

The configuration of the starch composite Sb accommodated in the accommodation section 52 is substantially the same as the configuration shown in FIG. 1A and therefore is not described. Incidentally, the starch composite Sba, which is shown in FIG. 1B, may be used instead of the starch composite Sb.

The configuration of each of the starch particles S1 and the second particles S2, which form the starch composite Sb, is substantially the same as the configuration shown in FIG. 1A and therefore is not described. The configuration of each of the starch particles S1 and the coat layers S2a, which form the starch composite Sba, is substantially the same as the configuration shown in FIG. 1B and therefore is not described.

In this embodiment, the accommodation section 52 is configured to accommodate the already formed starch composite Sb. The accommodation section 52 is not limited to this configuration. The accommodation section 52 may be connected to, for example, a manufacturing apparatus for manufacturing the starch composite Sb. That is, the accommodation section 52 may be connected to a high-speed rotary mixer. The accommodation section 52 is configured such that the starch particles S1 and the second particles S2, which contain the hydrophobic material, are charged into the high-speed rotary mixer, the starch composite Sb is manufactured by mixing the starch particles S1 and the second particles S2, and the manufactured starch composite Sb is supplied to the accommodation section 52. This enables the starch composite Sb to be supplied to the pipe 54 from the accommodation section 52 without stopping the operation of the fiber structure-manufacturing apparatus 1.

The starch composite Sb is melted by heating to bind a plurality of fibers. Thus, the starch composite Sb is in such a state that the starch composite Sb and the fibers Sa are mixed together. In such a state that the starch composite Sb is not heated to a temperature at which the starch composite Sb is melted, the fibers Sa are not bound.

The fragments P falling in the pipe 7 and the starch composite Sb supplied from the accommodation section 52 are sucked into the pipe 54 by a gas flow generated by the mixing blower 56 and pass through the inside of the mixing blower 56. The fibers Sa, which form the fragments P, are mixed with the starch composite Sb by the gas flow generated by the mixing blower 56 and the action of rotary portions such as blades included in the mixing blower 56. The mixture is transported to the accumulation section 60 through the pipe 54.

The accumulation section 60 imports the above mixture from an inlet 62, disentangles the intertwined fragments P, and sprays the fragments P such that the fragments P are dispersed in air. This enables the accumulation section 60 to uniformly accumulate the mixture on the second web-forming section 70.

The accumulation section 60 includes a drum portion 61 and a housing portion 63 housing the drum portion 61. The drum portion 61 is a cylindrical sieve rotationally driven with a motor. The drum portion 61 includes a net and functions as a sieve. Owing to meshes of the net, the drum portion 61 allows the fibers Sa and particles smaller than openings of the net to pass through and the fibers Sa and the particles fall from the drum portion 61. The configuration of the drum portion 61 is the same as the configuration of, for example, the drum portion 41.

The second web-forming section 70 is placed below the drum portion 61. The second web-forming section 70 accumulates a passing object having passed through the accumulation section 60 to form a second web W2 serving as a fibrous web. The second web-forming section 70 includes, for example, a mesh belt 72, a plurality of rollers 74, and a suction mechanism 76.

The mesh belt 72 is an endless belt, is tensioned with the rollers 74, and is moved by the motion of the rollers 74 in a direction indicated by an illustrated arrow. The mesh belt 72 is made of, for example, metal, resin, fabric, nonwoven fabric, or the like. A surface of the mesh belt 72 is composed of a net in which openings with a predetermined size are arranged. In the fibers Sa and particles falling from the drum portion 61, fine particles passing through meshes of the net fall below the mesh belt 72 and fibers incapable of passing through the meshes of the net accumulate on the mesh belt 72 and are transported in the direction of the arrow together with the mesh belt 72. The mesh belt 72 is moved at a constant speed V2 in usual operation for manufacturing the fiber structure S.

The meshes of the net of the mesh belt 72 are fine and may have a size not allowing most of the fibers Sa and particles falling from the drum portion 61 to pass through.

The suction mechanism 76 is placed below the mesh belt 72. The suction mechanism 76 includes a suction blower 77. The suction power of the suction blower 77 enables the suction mechanism 76 to generate a gas flow directed downward.

The mixture dispersed in air by the accumulation section 60 is sucked on the mesh belt 72 by the suction mechanism 76. This promotes the formation of the second web W2 on the mesh belt 72 and enables the discharge rate from the accumulation section 60 to be increased. Furthermore, a down-flow can be formed in the fall path of the mixture by the suction mechanism 76, thereby enabling the disintegrated matter and an additive to be prevented from being intertwined during falling.

As described above, passing through the accumulation section 60 and the second web-forming section 70 allows the second web W2 to be formed in such a state that the second web W2 contains a lot of air, is soft, and is bulgy. The second web W2 accumulated on the mesh belt 72 is transported to the fiber structure-forming section 80.

In a transport path of the mesh belt 72, air containing mist is supplied downstream of the accumulation section 60 by the humidification section 212. This supplies mist generated by the humidification section 212 to the second web W2 and adjusts the amount of moisture contained in the second web W2. This enables the adhesion of the fibers Sa to the mesh belt 72 due to static electricity to be suppressed.

The fiber structure-manufacturing apparatus 1 includes the transport section 79. The transport section 79 transports the second web W2 on the mesh belt 72 to the fiber structure-forming section 80. The transport section 79 includes, for example, a mesh belt 79a, rollers 79b, and a suction mechanism 79c.

The suction mechanism 79c includes a blower, which is not shown, and generates an upward gas flow by means of the suction power of the blower. This gas flow sucks the second web W2. The second web W2 is separated from the mesh belt 72 and is attracted to the mesh belt 79a. The mesh belt 79a is moved by the rotation of the rollers 79b to transport the second web W2 to the fiber structure-forming section 80.

As described above, the transport section 79 peels the second web W2 formed on the mesh belt 72 from the mesh belt 72 and transports the second web W2.

The fiber structure-forming section 80 forms the fiber structure S from accumulated matter accumulated by the accumulation section 60. In particular, the fiber structure-forming section 80 forms the fiber structure S by pressing and heating the second web W2 accumulated on the mesh belt 72 and transported by the transport section 79. In the fiber structure-forming section 80, the fibers Sa are bound with the starch composite Sb by heating the fibers Sa and starch composite Sb contained in the second web W2.

The fiber structure-forming section 80 includes a pressing portion 82 pressing the second web W2 and a heating portion 84 heating the second web W2 pressed by the pressing portion 82.

The pressing portion 82 is composed of a pair of calender rollers 85 and presses the second web W2 in such a manner that the second web W2 is nipped with a predetermined nip pressure. Pressing the second web W2 reduces the thickness of the web W and increases the density of the second web W2. One of the calender rollers 85 is a driving roller driven by a motor, which is not shown, and the other is a driven roller. The calender rollers 85 are rotated by the driving force of the motor and transport the second web W2 increased in density by pressing toward the heating portion 84.

The heating portion 84 is composed of, for example, a hot press molding machine, a hotplate, a hot air blower, an infrared heater, a flash-fusing system, an oven heater, a steam heater, a microwave heater, or the like. In the illustrated example, the heating portion 84 includes a pair of heating rollers 86. The heating rollers 86 are heated to a preset temperature by heaters placed inside or outside. The heating rollers 86 nip and heat the second web W2 pressed by the calender rollers 85, whereby the fiber structure S is formed so as to be strip-shaped.

One of the heating rollers 86 is a driving roller driven by a motor, which is not shown, and the other is a driven roller. The heating rollers 86 are rotated by the driving force of the motor and transport the heated fiber structure S toward the cutting section 90.

The cutting section 90 cuts the fiber structure S formed by the fiber structure-forming section 80. In the illustrated example, the cutting section 90 includes a first cutting portion 92 cutting the fiber structure S in a direction crossing the transport direction of the fiber structure S and a second cutting portion 94 cutting the fiber structure S in a direction parallel to the transport direction thereof. The second cutting portion 94 cuts the fiber structure S having passed through, for example, the first cutting portion 92.

The above allows the fiber structure S to be formed in the form of a single sheet with a predetermined size. The cut fiber structure S, which is such a single sheet, is discharged to a discharge section 96. The discharge section 96 includes a tray or stacker carrying the fiber structure S, which has a predetermined size.

Incidentally, the first cutting portion 92 and the discharge section 96 may be omitted. That is, the fiber structure S may be formed so as to be elongated and roll-shaped.

As described above, according to the fiber structure-manufacturing apparatus 1, since the starch composite Sb, which is supplied to the fibers Sa, is such that the surfaces of the starch particles S1 are covered by the second particles S2, which contain the hydrophobic material, the binding of the starch particles S1 is inhibited and the occurrence of aggregation can be suppressed. Since the aggregation of the starch particles S1 is suppressed, powder fluidity is enhanced. Therefore, the amount of the starch composite Sb supplied to the fibers Sa is constant. Furthermore, the supportability of the starch composite Sb on the fibers Sa can be enhanced, the binding of the fibers Sa can be increased, and the fiber structure S can be manufactured so as to have high quality.

In this embodiment, the starch composite Sb or Sba is used to manufacture the fiber structure S in a dry mode. The starch composite Sb or Sba is not limited to this and may be used to manufacture the fiber structure S in a wet mode. Even in this case, the starch particles S1 are unlikely to be thickened in a moist state and mixing, stirring, and kneading are easier in a wet mode.

### Examples

### Examples of the present disclosure are described below.

### 1. Examples 1 to 4

As shown in Table 1, starch and a hydrophobic material were mixed together, whereby a starch composite was manufactured. Thereafter, the starch composite and cellulose were formed into a web by an air-laid process. Thereafter, the web was heated and was pressed, whereby a sheet-shaped fiber structure was formed.

### 2. Comparative Example 1

As shown in Table 1, starch and cellulose were formed into a web by the air-laid process. Thereafter, the web was heated and was pressed, whereby a sheet-shaped fiber structure was formed.

### 3. Evaluations

Powder fluidity evaluation, supportability evaluation, and uniformity evaluation were performed.

### 3-1. Powder Fluidity Evaluation

Powders of the starch composites manufactured in Examples 1 to 4 and the starch used in Comparative Example 1 were evaluated for fluidity.

In particular, each starch composite powder or the starch powder was dropped from a certain height toward a measurement stage and the angle (angle of repose) formed by a slope of a pile of the starch composite or starch powder and the horizontal plane was measured with a protractor when the pile did not collapse spontaneously but remained stable.

### 3-1-1. Evaluation Standards

A: An angle of repose of less than 40°.
B: An angle of repose of 40° to less than 50°.
C: An angle of repose of 50° or more.

### 3-2. Supportability Evaluation

In each of Examples 1 to 4, the remaining amount of the starch composite in the fiber structure with respect to the adhesion amount of the starch composite contained in the web was measured.

In Comparative Example 1, the remaining amount of the starch in the fiber structure with respect to the adhesion amount of the starch contained in the web was measured.

The adhesion amount and remaining amount of each of the starch composite and the starch were measured on the basis of observation using an optical microscope (VHX-5000 manufactured by Keyence Corporation, 300x to 500x magnification). An evaluation sample was such that starch was dyed with an iodine-potassium iodide solution in advance. An image obtained by observation was binarized and the image area ratio after dyeing was measured. 3-2-1. Evaluation Standards
A: A remaining amount of 80% or more.
B: A remaining amount of 60% to 79%.
C: A remaining amount of 59% or less.

### 3-3. Uniformity Evaluation

In each of Examples 1 to 4, the relative ratio of the adhesion amount of the starch composite on a first surface of the fiber structure to the adhesion amount of the starch composite on a second surface opposite to the first surface was measured.

In Comparative Example 1, the relative ratio of the adhesion amount of the starch on a first surface of the fiber structure to the adhesion amount of the starch on a second surface opposite to the first surface was measured.

The adhesion amount of the starch composite or the starch was measured on the basis of observation using an optical microscope (VHX-5000 manufactured by Keyence Corporation, 300x to 500x magnification). An evaluation sample was such that starch was dyed with an iodine-potassium iodide solution in advance. An image obtained by observation was binarized and the image area ratio after dyeing was measured.

### 3-3-1. Evaluation Standards

A: A relative ratio of 100:100 to 100:80.
B: A relative ratio of 100:79 to 100:60.
C: A relative ratio of 100:59 or less.

Results are as shown in Table 1.

**Table 1**

| | Starch composite | | | Fiber structure | | | Evaluations | | |
|---|---|---|---|---|---|---|---|---|---|
| | Binder | Hydrophobic material | Stirring treatment | Fiber | Web-forming treatment | Heat pressing treatment | Powder fluidity evaluation | Supportability evaluation | Uniformity evaluation |
| Example 1 | 39.7g of starch | 0.79g of stearic acid | Air stirring | 59.5g of cellulose | Air-laid | 150°C for 1 min | B | B | B |
| Example 2 | Ditto | Ditto | High-speed mixer, 20,000 rpm for 1 min | Ditto | Ditto | Ditto | A | A | A |
| Example 3 | Ditto | 0.80g of shellac | Ditto | Ditto | Ditto | Ditto | A | A | A |
| Example 4 | Ditto | 0.79g of stearic acid | Ditto | Ditto | Ditto | Water mist (adding 20% of weight of web, followed by heating at 150°C for 1 min) | A | A | A |
| Comparative Example 1 | Ditto | Not used | - | Ditto | Ditto | 150°C for 1 min | C | C | C |

As shown in Table 1, in Examples 1 to 4, excellent results were obtained for powder fluidity, supportability, and uniformity. That is, in a starch composite, the aggregation of starch particles is suppressed and a failure such as a rathole is reduced; hence, the starch composite can be stably supplied to fibers. The starch composite is likely to be supported between the fibers, leading to the increase in binding strength of the fibers. Furthermore, the starch composite is uniformly dispersed in a fiber structure and therefore the uniformity of the binding strength between the fibers can be ensured.

However, it was clear that Comparative Example 1 was poorer in all evaluations as compared to Examples 1 to 4.

## Claims

1. A starch composite for binding fibers, comprising:
starch particles which are first particles; and
second particles containing a hydrophobic material having an affinity for the starch particles, which are the first particles, wherein
the weight-average size of the second particles is less than the weight-average size of the first particles and the outer surfaces of the first particles are covered by the second particles.

2. The starch composite according to claim 1, wherein the second particles are composed of one or a combination of two or more of hydrocarbons, fatty acids, fatty acid metal salts, higher alcohols, aliphatic amides, fatty acid esters, and inorganic materials.

3. A starch composite for binding fibers, comprising:
starch particles; and
coat layers which are made of a hydrophobic material having an affinity for the starch particles and which coat the starch particles.

4. The starch composite according to claim 3, wherein the coat layers are composed of one or a combination of two or more of hydrocarbons, fatty acids, fatty acid metal salts, higher alcohols, aliphatic amides, fatty acid esters, and inorganic materials.

5. A fiber structure containing:
cotton-like fibers;
starch particles; and
a hydrophobic material having an affinity for the starch particles, wherein
the starch particles and the hydrophobic material are dispersed between the cotton-like fibers and the cotton-like fibers, the starch particles, and the hydrophobic material are bound.

6. The fiber structure according to claim 5, wherein particles of the hydrophobic material have a weight-average size less than the weight-average size of the starch particles and cover the surfaces of the starch particles.

7. The fiber structure according to claim 5, further comprising coat layers which are made of the hydrophobic material and which coat the starch particles.

8. The fiber structure according to claim 5, wherein the hydrophobic material is composed of one or a combination of two or more of hydrocarbons, fatty acids, fatty acid metal salts, higher alcohols, aliphatic amides, fatty acid esters, and inorganic materials.

9. A fiber structure-manufacturing apparatus comprising:
a disintegration section disintegrating a supplied fiber feedstock to produce disintegrated matter;
an incorporation section incorporating a starch composite having a surface covered by a hydrophobic material having an affinity for starch particles into the produced disintegrated matter;
a fibrous web-forming section forming a fibrous web by accumulating a mixture containing the starch composite; and
a fiber structure-forming section forming a fiber structure by heating and pressing the fibrous web.

10. The fiber structure-manufacturing apparatus according to claim 9, wherein the hydrophobic material is composed of one or a combination of two or more of hydrocarbons, fatty acids, fatty acid metal salts, higher alcohols, aliphatic amides, fatty acid esters, and inorganic materials.
